# EUROPEAN PATENT SPECIFICATION

(11) **EP 1 561 436 B1**
(45) Date of publication and mention of the grant of the patent: **08.08.2007**
(21) Application number: 05001267.3
(22) Date of filing: 21.01.2005
(51) Int. Cl.: A61F 2/82

(54) **A stent for endoluminal delivery of active principles or agents**
Stent für die Freisetzung von aktiven Substanzen
Stent pour la libération d'un principe actif

(30) Priority: 05.02.2004 IT TO20040056
(43) Date of publication of application: 10.08.2005
(73) Proprietor: SORIN BIOMEDICA CARDIO S.R.L., 13040 Saluggia (Vercelli) (IT)
(72) Inventor: Curcio, Maria, 13040 Saluggia (Vercelli) (IT); Pasquino, Enrico, 10020 Marentino (Torino) (IT); Rolando, Giovanni, 10034 Chivasso (Torino) (IT); Grignani, Andrea, 10023 Chieri (Torino) (IT)
(74) Representative: Freyria Fava, Cristina

(56) References cited:
- EP-A- 1 449 546
- WO-A-97/33552
- WO-A-02/065947
- WO-A-20/04105646
- WO-A-20/04110347

## Description

The present invention relates to endoluminal delivery of active principles or agents.

The invention has been developed with particular attention paid to its possible application to stents, where said term usually identifies expandable endoprostheses that can be implanted in a lumen of the human or animal body, such as for example a blood vessel, to re-establish and/or maintain patency thereof.

Stents are usually configured as devices comprising a tubular body which operate so as to maintain a segment of a blood vessel or of another anatomical lumen open.

Stents have particularly asserted their presence in the course of the last few years for use in the treatment of stenosis of an arteriosclerotic nature in blood vessels, such as the coronary arteries. A similar process can find application also in the dilation of the carotid or peripheral arteries.

The scientific and technical literature, including the patent literature, regarding stents is extremely extensive.

Just to limit our examination to the documents filed in the name of the present applicant, reference may, for example, be made to the documents Nos. EP-A-0 806 190, EP-A-0 850 604, EP-A-0 875 215, EP-A-0 895 759, EP-A-0 895 760, EP-A-1 080 738, EP-A-1 088 528, EP-A-1 103 234, EP-A-1 174 098, EP-A-1 212 986, EP-A-1 277 449, EP-A-1 310 242, as well as to the European patent application No. 03425106.6 (published as EP-A-1 449 546 on 25.08.2004 with a priority date of 21.02.2003).

The activities of research, development and industrial production of stents were directed in the first few years principally to the geometrical structure and to the corresponding techniques of fabrication (winding of a wire, cutting starting from a microtube, use of superelastic materials, etc.).

The research activity regarding stents has then gradually extended to other particularities of their fabrication, and in particular to the possibility of applying on the stent or, in some way, associating to the stent, substances having the nature of drugs, and hence able to perform a specific activity on the site of implantation of the stent.

Solutions of this nature are described, in the framework of the documents cited above, in EP-A-0 850 604, EP-A-1 080 738 and EP-A-1 103 234.

The document No. EP-A-0 850 604 describes the possibility of providing the stent with grooving comprising, for example, cavities that may receive one or more drugs useful in the prevention or in the treatment of restenosis, and/or other substances appropriate for a correct use of the stent (adhesion, modalities of release of the active principle, kinetics, etc.). Said surface grooves are characterized, as regards the boundary, as regards the surface of the cavity, and as regards the profile in depth. For example, the cavities can be cavities with circular or else ovoid or else elongated openings. Alternatively, they can assume the form of an appropriate alternation of cavities with openings of different types according to the requirements of drug release. The depth profile can be U-shaped or else V-shaped, or else shaped like a vessel, with or without a surface part completely dedicated to receiving the substances of interest referred to above. Said surface part can assume the appearance of a sort of continuous layer on just the outer surface of the stent.

A solution of this type is adopted also in WO-A-98/23228, in EP-A-0 950 386 and again in the European patent application No. EP-A-1 277 449.

The solution described in the latter application envisages that in the elements of the reticular structure of the stent there will be provided cavities that can function as true reservoirs for receiving agents for the treatment of the site of implantation of the stent. Where present, the cavities bestow upon the respective element a hollowed profile, the cavities occupying a substantial portion thereof. The geometry of the cavities is chosen in such a way as to leave substantially unimpaired the characteristics of resistance to bending of the respective element.

Said solution enables the quantity of agent associated to the stent to be in any case sufficient even when it is desired to obtain a release, and hence an action, that is prolonged in time, also considering the fact that, in applications of angioplasty, the surfaces of the stent, and above all the internal surface, are subjected to an action of flushing by the blood flow.

Furthermore, this solution enables the active or activatable agent to be made available and released prevalently, if not exclusively, on the outer surface of the stent and not on the internal surface thereof. This applies above all in the case where the agent applied on the stent has to perform a restenosis-antagonist function. The corresponding mechanism of action, which aims at acting on the outer surface of the stent facing the wall of the treated vessel, could in fact have adverse effects on the internal surface: for example, it could hinder phenomena of formation of neointima on the internal surface of the stent, which are considered certainly beneficial in the post-implantation phase.

The same solution then makes it possible to have available stents capable of being configured as true vectors of active or activatable agents, possibly different from one another, made available in sufficient quantities to achieve a beneficial effect that is also prolonged in time. Added to the above is the further possibility of having available agents that may even be different from one another, located selectively in different positions along the development of the stent, so as to be able vary selectively the dosages in a localized way, for example achieving differentiated dosages in the various regions of the stent.

The solutions described in the patent documents cited previously respond primarily to requirements linked to the mechanism of release of the active agent. This in particular as regards: i) the quantity of agent that may be released, ii) the position in which the agent (or the various agents) arranged on the stent are released, and, albeit in to a lesser extent, iii) the kinetics of release of the active agent.

The present invention belongs within the line of research aimed at the identification of pharmacologically active compounds, or, again more preferably, of associations of pharmacologically active compounds to be vehicled via a stent and that are to perform an effective restenosis-antagonist function.

It has in fact been established that, in a relatively large proportion of patients to which a stent had been applied, there developed a new stenosis. It has been discovered that this so-called restenosis is generated by a new formation of the vascular architecture of the layers of tissue. In particular, the introduction of a stent in the stenotic site entails damage to the tissues of the blood vessel (generally referred to as "mechanically mediated vascular injury") with consequent inflammatory reactions, hyperproliferation, and migration of smooth muscle cells (SMCs) into the damaged stenotic site.

In the model of animal restenosis and also in human tissue it has been found that the hyperproliferation of SMCs is accompanied by infiltration of the tissue around the reinforcements of the stent by macrophages and T-cells, as is for example described in Grewe et al., J. Am. Coll. Cardiol. 35 (2000), 157-63.

Research activity then concentrated on the identification of pharmacologically active substances, in particular with the aim of providing a stent with a capacity for release *in situ* of one or more of these substances, exploiting possible synergistic or diversified reactions. Specific attention has been paid to the role played by certain drugs in regard to inflammatory and/or hyperproliferative reactions. In addition to pharmacologically active molecules, the stent can carry substances with a function of adjuvant of the pharmacologically active substances, such as polymers or excipients of various nature. The function of the latter may be stablization of the active principles, or else be aimed at regulating the kinetics of release (deceleration or acceleration of release). The polymers/excipients can be mixed with the drug or drugs or else be in separate layers with respect to the pharmacologically active substances, for example forming in the context of a cavity a sort of operculum of bio-erodible polymer, i.e., creating a stratified structure, with successive layers of drug and polymer.

Also in relation to these aspects, the technicoscientific and patent literature is extremely extensive, as witnessed, not only by some of the documents already cited previously, but also by documents such as, for example, Nos. EP-A-0 551 182, EP-A-0 747 069, EP-A-0 950 386, EP-A-0 970 711, EP-A-1 254 673, EP-A-1 254 674, WO-A-01/87368, WO-A-02/26280, WO-A-02/26281, WO-A-02/47739, WO-A-02/056790, and moreover WO-A-02/065947, WO-A-01/87372, WO-A-01/87375, WO-A-95/03036, as well as by the literature cited in said documents; documents and literature which, of course, certainly do not exhaust the panorama on the subject.

As regards the choice of the drug with restenosis-antagonist function, the drugs proposed for said purpose are extremely numerous, and even more numerous - even though not fully described - are the hypothetical combinations of two or more drugs belonging to the same class or to different classes. The drugs proposed comprise, for example: *anti-inflammatory drugs of the corticosteroid type* (Cortisol, Betamethasone, Fluocinolone, Cortisone, Dexamethasone, Fluocinonide, Corticosterol, Flunisolide, Fluoromethalone, Tetrahydrocortisol, Alclomethasone, Flurandrenolide, Prednisone, Amcinonide, Alcinonide, Prednisolone, Clobetasol, Medrisone, Methylprednisolone, Clocortolone, Momethasone, Fluodrocortisone, Desonide, Rofleponide, Triamcinolone, Desoxymethasone, Paramethasone, Diflorasone); *anti-inflammatory agents of the non-steroidal type* (Acetylsalicylic acid, Diflunisal, Salsalate, Phenylbutazone, Oxyphenbutazone, Apazone, Indomethacin, Sulindac, Mefenamic acid and fenamates, Tolmetine, Ibuprofen, Naproxen, Fenoprofen, Ketoprofen, Flurbiprofen, Piroxicam and derivatives, Diclofenac and derivatives, Etodolac); and *anti-neoplastic, anti-proliferative, and*/*or immunosuppressive agents* (Cyclophosphamide, Melphalan, Chlorambucil, Ethyleneimine and Methylmelamine, Alkyl sulphonates, Nitrosoureas, Triazines, Metotrexate, Fluorouracil, Mercaptopurine, Thioguadinine, Vinblastine, Vincristine, Etoposide, Actinomycin D, Doxorubicin, Cisplatin, Mitoxantrone and derivatives, Hydroxyurea and derivatives, Procarbatine and derivatives, Mitotanes, Aminoglutetimide, Docetaxel, Paclitaxel and analogues, 7-hexanoyl-taxol, Epothilones, Batimastat and analogues, Rapamycin and analogues, FK506 (tacrolimus), Cyclosporine).

Some of the compounds cited above have constituted the subject of patent publications; in particular, there are cited the patent applications Nos. WO-A-02/065947 and EP-A-1 254 674 regarding the use of FK506 (an immunosuppressive drug) and the applications Nos. WO-A-01/87372, WO-A-01/87375 and WO-A-95/03036 regarding the use of Paclitaxel (anti-proliferative agent).

The application No. WO-A-02/065947 discloses a stent according the preamble of claim 1. This stent is loaded with FK506 possibly in combination with other active substances, where the list of the possible additional drugs comprises approximately sixty compounds. The modalities of application of the drug to the stent envisage that the stent will be brought into contact with a solution of FK506 in aqueous or organic solvent (typically in alcohol) for example, by means of dripping, spraying, or immersion, preferentially under a vacuum. The stent is then dried, preferably up to total removal of the solvent, the operation being repeated from 1 to 5 times. Subsequently, the stent is possibly washed with water or isotonic saline solution and then dried again.

It has, however, been noted that the technical solution described in the field of the aforesaid patent application may not be effective in the treatment of restenosis. It may be hypothesized that this derives from the fact that the amount of drug, and in particular of FK506, loaded on the stent following the method described herein, is not pharmacologically active in regard to the cell processes that underlie restenosis, presumably in so far as it is smaller than the threshold value of therapeutic effectiveness.

This result is corroborated also by the scientific literature that has experimented FK506 in cultures of SMCs in view of inhibition of the proliferation of smooth muscle cells (Mohacsi et al., J. Heart Lung Transplant. 16 (1997) 484-492; Marx et al., Circulation Res., 76 (1995) 412-417), and of their migration (Poon et al., J. Clin. Invest. 98 (1996) 2777-2283). In general, FK506 has been deemed unsuitable for the prevention of restenosis on account of its low power, as witnessed by Mohacsi et al. J. Heart Lung Transplant. 16 (1997) 484-492; Poon et al. J. Clin. Invest. 98 (1996) 2777-2283; Marx et al., Circulation Res., 76 (1995) 412-417; Dell, Curr. Med. Chem. 5 (1998) 179-94. Rapamycin (another immunosuppressive drug) has proven, instead, active in the inhibition of the proliferation of SMC cultures.

Once again in relation to the application of FK506, it is possible to cite the European patent application No. EP-A-1 254 674, in the framework of which are identified quantities by weight of FK506 usable on a stent as restenosis-antagonist agent. This description is, however, highly ambiguous in so far as the quantities indicated of a drug to be used are alternatively expressed in milligrams, micrograms and picograms, always in relation to a stent of 16 mm in length. Such a description does not therefore indicate a therapeutically effective concentration of FK506 as agent restenosis-antagonist, nor does it enable such a concentration to be deduced.

The documents Nos. WO-A-01 87372, WO-A-01 87375 and WO-A-95 03036 describe the use of the anti-proliferative drug identified as Paclitaxel or Taxol, a substance with cytostatic, anti-proliferative, and/or anti-angiogenic activity, applied directly or indirectly on a stent, once again for the purpose of acting as restenosis-antagonist.

Also in relation to the use of said drug, possibly in combination with another pharmacologically active compound, such as for example Rapamycin, there exist numerous scientific articles (Herdeg et al., Semin. Intervent. Cardiol. 3 (1998) 197-199; Hunter et al., Adv. Drug Delivery Rev. 26 (1997) 199-207; Burke et al., J Cardiovasc. Pharmacol., 33 (1999) 829-835; Gallo et al., Circulation 99 (1999) 2164-2170), according to which the results of the application of the association of Paclitaxel in combination with Rapamycin in laboratory animals is not effective in the treatment of restenosis and even harmful in so far as it would seem to lead to inhibition of the formation of neointima. It has moreover been observed that after six months from insertion in pigs of stents coated with Paclitaxel, there was found a disappearance of the effect (Heldman, International Local Drug Delivery Meeting and Cardiovascular Course on Radiation, Geneva, Jan 25-27, 2001).

So far Rapamycin proves - according to prevalent opinion - the drug with the best potentiality of application for an almost complete elimination of restenosis, as supported by the first clinical findings (Sousa et al., Circulation 103 (2001) 192-195). On the other hand, the use of the Rapamycin - according to some scientists - would seem to give rise to a decelerated healing of the vascular wall injured by balloon angioplasty and insertion of the stent.

It is thus very important to achieve a balance between treatment of the arterial vascular wall after angioplasty and insertion of the stent, on the one hand, and the formation of neointima on the internal wall of the stent facing the blood flow, on the other.

The task of the present invention consists in making available implants with properties favourable for the treatment of restenosis and capable of reducing undesirable effects to the minimum.

According to the present invention, said task is achieved thanks to a stent having the characteristics referred to specifically in the ensuing claims.

In summary, the solution described herein is based upon the observation of the fact that the target of a truly effective restenosis-antagonist action can be achieved via identification of a combination of drugs and more preferably through identification of the correct ratios (concentrations) between the two drugs of the combination so as to reduce to the minimum their secondary effects and increase as much as possible their pharmacological activity.

Furthermore, the present applicant has verified that the modalities of loading of the association of drugs on the stent are important. It is in fact preferable that the drugs should be free, i.e., that they should be applied without prior dissolution or suspension as commonly envisaged in the known art, where techniques of dripping, immersion, or spraying, on the stent, of a solution containing the active principle dissolved in a solvent are adopted.

The subject of the present invention is a stent to which there are associated Paclitaxel and FK506 or their derivatives or analogues in combination, appropriately loaded within the cavities present on the outer surface of the stent, and optionally also on the entire outer surface of the stent, in a Paclitaxel:FK506 weight ratio with respect to the total quantity of said agents loaded on the stent comprised in the range between 1:72 and 1:0.2.

The invention will now be now described in a detailed way, purely by way of non-limiting example, with reference to the annexed drawings, in which:
- Figure 1 is a schematic illustration of the cross section of a stent usable in the context of the present invention;
- Figures 2 to 7 are schematic illustrations of the modalities of embodiment of the solution according to the invention; and
- Figure 8 illustrates the embodiment of an operation of loading of a stent using a device for dispensing a paste.

The present description is provided, purely by way of non-limiting example, with reference to a stent 1 substantially corresponding to the stent described in the document No. EP-A-0 875 215.

Such a stent is constituted by a tubular body made of metallic material, that may be dilated starting from a radially contracted condition into a radially expanded condition.

The body of the stent comprises a plurality of structural elements or "struts" 10, which define an openwork structure as a whole of reticular nature.

In particular, in the solution described in the document No. EP-A-0 875 215, the structure in question comprises a plurality of annular segments arranged in succession along the longitudinal axis of the stent. The segments in question have a serpentine pattern with the bend parts arranged in opposite sequence, connected together by connecting elements, commonly referred to as "links". The serpentine sequences of the successive segments are usually in phase opposition, i.e., with a concavity of each serpentine opposite to a concavity of an adjacent segment.

The links have a substantially V-shaped pattern with a profile characterized by an alternation of concave portions and convex portions. The aforesaid links connect the various annular segments of the stent at the "0" points of the serpentine paths of the segments.

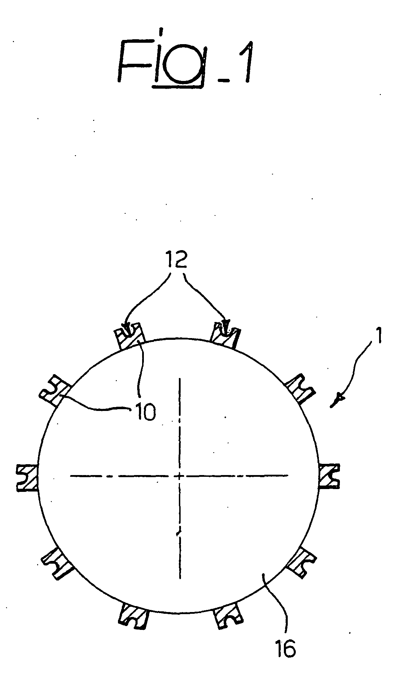

In the view of Figure 1 the stent 1 is represented in cross section, so that there appears only the circular trace of cross section defined by a certain number of struts 10 traversed by the plane of cross section.

The stent in question is provided on its outer surface with grooving formed by a pattern with cavities 12 of the type described in EP-A-0 850 604, or else in the European patent application No. EP-A-1 277 449.

The cavities 12 can receive within them respective amounts of a substance constituted by Paclitaxel and FK506, according to the modalities described in what follows.

In a particularly advantageous way, the stent in question is coated on its outer surface by a layer of biocompatible carbon material deposited thereon, by applying the technique described, for example, in the documents Nos. US-A-5 084 151, US-A-5 133 845, US-A-5 370 684, US-A-5 387 247 and US-A-5 423 886.

The presence of said coating of biocompatible carbon material proves advantageous for the purposes of use of the stent, in particular as regards the minimization of the effects mediated by implantation of the stent.

In summary, the solution described herein envisages carrying out the loading/application on a stent starting from powders or pastes obtained from the powders of the two drugs Paclitaxel and FK506 possibly mixed together, where the two drugs have a Paclitaxel:FK506 weight ratio - with respect to the total quantity of drugs loaded on a stent - comprised in the range from 1:72 to 1:0.2, preferably from 1:18 to 1:0.2, more preferably from 1:8 to 1:0.5, still more preferably from 1:2 to 1:0.7, and to a more preferred extent from 1:3 to 1:1.

With reference to assessments regarding a stent for coronary angioplasty having a length of approximately 15 mm and a diameter in the expanded condition of approximately 3-3.5 mm, the present applicant has reason to believe that a total quantity of drugs, Paclitaxel and FK506, loaded/applied on the stent - without considering the possible presence of additives and/or excipients - comprised in the range between 50 and 1000 micrograms may be therapeutically effective. The total quantity of Paclitaxel and FK506 loaded/applied on the stent can be to a more advantageous extent comprised in the range between 70 and 700 micrograms, preferably said quantity may be comprised between 170 and 500 micrograms, to a still more preferred extent between 200 and 400 micrograms. It is evident that when reference is made to loading/application on the stent of said drugs, this may mean both the loading within the cavities alone present on the outer surface of the stent and a generalized loading on the entire outer surface of the stent, including loading also within the cavities.

The present applicant, once again on the basis of evaluations referring to a stent for coronary angioplasty having a length of approximately 15 mm and a diameter in the expanded condition of approximately 3-3.5 mm, has moreover reason to believe that the minimum therapeutically effective quantity of Paclitaxel, when this drug is in combination with FK506, is greater than 25 micrograms and more preferably greater than 80 micrograms.

Albeit without wishing to be tied down to any theory in this regard, it is believed that the therapeutic effectiveness of the one active agent is supported rather than integrated by the presence of the other active agent comprised in the combination, even though the other active agent is present in a minimum amount and possibly less than the threshold value of therapeutic effectiveness reported in the literature. It may be hypothesized, in fact, that in these conditions the combined therapeutic effect of the two drugs is exploited directly on the stenotic site, i.e., the direct application of the two drugs on the stenotic blood vessel, in which implantation of the stent has been performed to re-establish and/or maintain its patency, will determine a tissue concentration of the two drugs sufficient to achieve therapeutic effectiveness.

In a way similar to what has been described above in relation to Paclitaxel., the present applicant has reason to believe that the minimum amount of FK506 therapeutically effective in the stenotic site, when said drug is applied in combination with Paclitaxel, is greater than 60 micrograms and more preferably greater than 135 micrograms.

It should be recalled that FK506 is the macrolide antibiotic FK506 (Tacrolimus, [3S-[3R*[E(1S*,3S*,4S*)],4S*, 5R*,-8S*,9E,12R*,14R*,15S*,16R*,18S*,19S*, 26aR*]]-5,6,8,11,12,13,14,15,16,17,18,19,24,25,25,26a-hexadecahydro-5,19-dihydroxy-3-[2-(4-hydroxy-3-methoxycyclohexyl)-1-methylethenyl]-14,16-dimethoxy-4,10,12,18-tetramethyl-8-(2-propenyl)-15,19-epoxy-3H-pyrido[2,1-c][1,4]oxa-aza-cyclotricosine-1,7,20,21(4H,23H)-tetrone; Merck index No. 9000).

It is an active substance which was originally developed for the medicine of transplants and the immunosuppressive action of which is considered to extend also to the restenosis-antagonist mechanism.

Paclitaxel belongs to the family of the diterpenoids (Taxol, [2aR- [2aα,4β,4aβ,6β,9α-(αR*,βS*),11α, 12α,12aα,12bα]]-β-(benzoylamino)-α-hydroxy-benzene propanoic acid 6,12-bis(acetyloxy)-12-(benzoyloxy)-2a,3,4,4a,5,6,9,10, 12,12a,12b-dodecahydro-4,11-dihydroxy-4a,8,13,13-tetramethyl-5-oxo-7,11-methano-1*H*-cyclodeca-[3,4]benz[1,2-*b*]oxet-9-yl ester; Merck index No. 7052); Paclitaxel is extracted from the bark of *Taxus brevifolia* and is produced by *Taxomyces Andreanae*, an endophytic fungus of *Taxus brevifolia* (Stierle et al., Science 260, 214-216, 1993). Paclitaxel is distributed commercially under the trade name Taxol® by Brystol Meyers Squibb.

Paclitaxel exerts an anti-proliferative and anti-angiogenic effect by acting on the intracellular microtubules.

Albeit without wishing to be tied down to any specific theory in this regard, the present applicant has reason to believe that the use of the association of the two drugs or of their derivatives or analogues, above all in the particular conditions of application, i.e., of concentration of the two drugs with respect to one other, will lead to Paclitaxel being able to act as inhibitor of proliferation of SMCs, altering the dynamic balance between microtubules and α- and β-tubulin, favouring the formation of abnormally stable microtubules. Paclitaxel in this way interferes with the capacity of the cell to maintain its shape and directionality of movement, to transmit intracellular signals and to carry out intracellular transport. FK506 is believed to be able to explicate its immunosuppressive action by contrasting the presence of macrophages and T-cells in the implantation site, and it is also believed that it may be able to act as anti-inflammatory agent, mitigating the inflammatory action induced by the procedure of angioplasty and insertion of the stent, enabling the normal phenomenon of healing/cicatrization of the wall of the vessel.

It has moreover been verified that the application in the form of powders or of pastes formed from said powder manages to reconcile the need to provide a selective application (an important factor both for the purposes that it is intended to pursue and for the specific cost, which is usually rather high, of the substances applied), with the need to ensure firm retention of the substances applied on the stent. The specific details of the application of said powders or pastes are described in the patent application No. 03425106.6 (published as EP-A-1 449 546 on 25.08.2004 with priority date of 21.02.2003) in the name of the present applicant, and which currently constitutes a document of the prior art in conformance with Art. 54(3) EPC.

It will be appreciated that the term "paste" is intended herein to indicate any plastic mass having a degree of viscosity such as to cause said mass to conserve substantially its shape if exposed only to the force of gravity.

The above is in evident contrast with a liquid or cream, which does not present said characteristic of conservation of shape.

The solution described herein envisages that there may be added to the two drugs, either mixed therewith or in separate layers, excipients and/or polymers having the purpose of agents for filling the cavities 12 set on the outer surface of the stent or of agents having the capacity of controlling (i.e., accelerating or decelerating) release of the drugs.

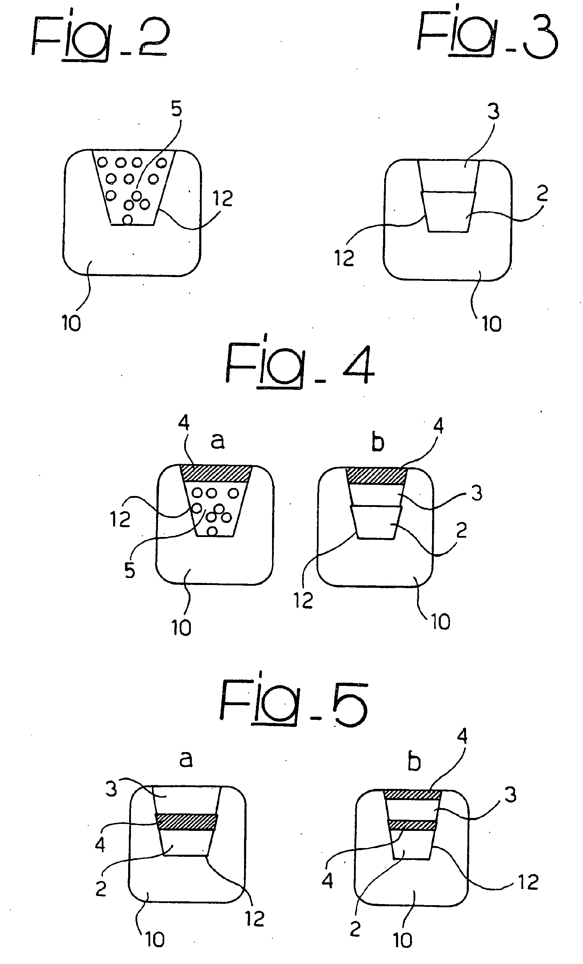

An embodiment of the solution described herein is illustrated in Figure 2, which shows just one strut provided with a cavity 12 that is to be filled with a mixture of the two drugs 5, where the mixture 5 is constituted by Paclitaxel and FK506 in a weight ratio with respect to the total amount of drugs loaded on the stent - without considering the presence of possible excipients and/or additives - comprised between 1:72 and 1:0.2, advantageously between 1:18 and 1:0.2, more preferably between 1:8 and 1:0.5, still more preferably between 1:2 and 1:0.7, and to an even more preferred extent between 1:3 and 1:1.

This embodiment exploits the effect linked to the simultaneous release of the two drugs. The mixture, as already anticipated previously, can contain possible excipients.

The process of loading of the mixture can be selective in the sense that it concerns only those areas of the stent that effectively carry the drugs, as well as the possible additives (excipients).

Of course, this result can be achieved also by way of a generalized loading, initially involving also areas of the stent that are then to be cleaned from the excess of the substances loaded.

Once the chosen substances have been deposited on the stent (before or after cleaning, according to the operating choices or the techniques adopted), the next step is fixation (stablization) of the drugs, as well as of the additives. The purpose of this is to ensure that said substances may be effectively vehicled by the stent onto an implantation site and not be dispersed elsewhere, in particular during advance towards the implantation site or even before the stent is inserted into the body in which it is to be implanted.

At least in principle, the operations of stablization can be conducted in a selective way only on the parts where there is required the presence of the substances deposited or else in a generalized way on the entire stent, at least as regards the outer surface thereof.

Specifically, in the case where the process starts from powders, the main methods of loading that can be adopted are:
- recourse to a non-selective corona effect (electrostatic effect), i.e., coating all the surface with powder and then cleaning the areas that require cleaning;
- recourse to a selective electrostatic process (such as a photocopier or laser printer), for example through an intermediate roller that collects the powder only in areas corresponding to the cavities where it deposits the powder subsequently; if the stents are ones having the entire outer surface porous, in general selectivity is not necessary, and no specific operation of cleaning may be required;
- rolling on a bed or mat of powder, with subsequent cleaning; and
- rolling on a bed or mat of powder with a mask of a serigraphic type so as to load just the cavities; cleaning is not required.

In the case where the process starts from a paste, the main methods of loading that can be adopted are:
- rolling on a bed or mat of paste, with subsequent cleaning;
- rolling on a bed or mat of paste with a protective mask (serigraphy); cleaning is not required; and
- application with a dispensing nozzle, typically driven by a fine-positioning numeric-control machine.

It will moreover be appreciated that whatever the modalities of loading adopted, for pastes or for powders, it is then preferable to proceed to an operation of stablization, where by the term "stablization" is of course meant bestowing upon the contents of the cavities a mechanical stability and a degree of adhesion to the cavities themselves adapted to the mechanical stresses undergone by the stent during transportation, stockage, procedures of insertion into the body, and expansion.

To achieve the desired effect of stablization it is possible to resort to techniques such as:
- exposure to temperature or thermal cycles;
- immersion in solvent for controlled lengths of time;
- exposure to solvent sprays;
- exposure to solvent vapours;
- selective treatment with laser (exposure of the cavities to a laser beam);
- selective or integral application of an adhesive protective coating (as an operculum for closing the cavities); and
- liophilization.

It will likewise be appreciated that the final result achieved is the placing of the drug or of the mixture on the surface of the stent 1, in a position directly exposed on the outside, even though usually at least slightly retracted inside the cavities 12, without any need to have available coatings/sheaths of some kind on the surface of the stent. The latter thus remains free, with the coating of biocompatible carbon material preferentially provided thereon, with consequent beneficial effects both during implantation of the stent and in the post-implantation phases.

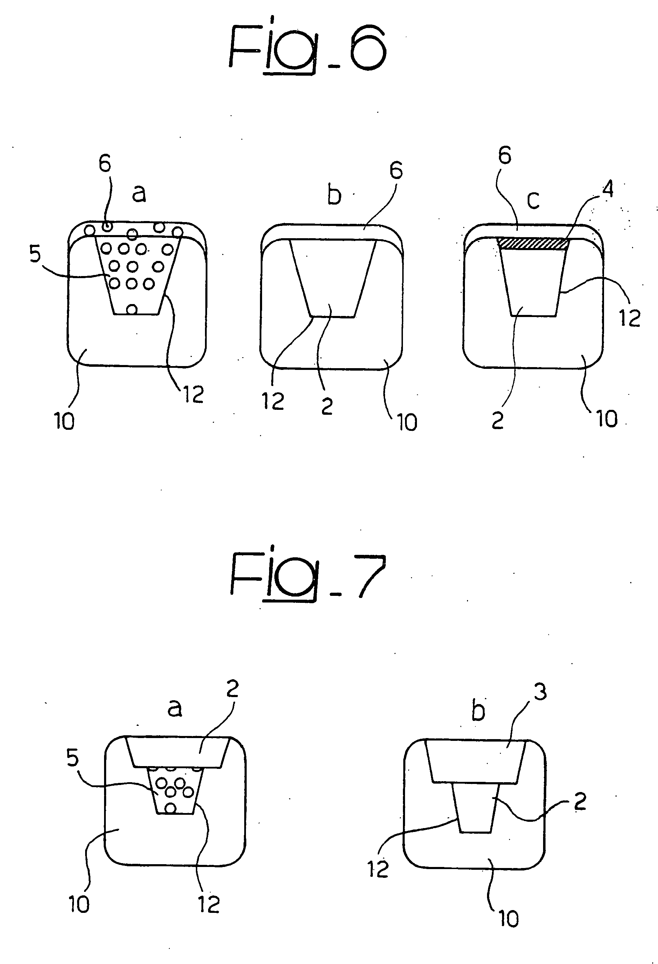

A second example of embodiment of the solution described herein is represented in Figure 3, where the cavity 12 can be filled - according to the modalities described above - in two steps, setting on the bottom of the cavity 12 a first layer 2, constituted by Paclitaxel, and on top a second layer 3, constituted by. FK506, or else reversing the composition of the first layer and the second layer so as to have a first layer 2 of FK506 on the bottom of the cavity 12 and on top a second layer 3 of Paclitaxel, or again combining a first layer 2 of a drug (whether it be Paclitaxel or FK506) with a second layer 3 constituted by a mixture of the two drugs or vice versa (layer 2 constituted by a mixture, and layer 3 constituted by a drug), or again both of the layers 2 and 3 constituted by mixtures of the two drugs, but with ratios different from one another, the foregoing once again in due respect of the ratios/quantities by weight between the two drugs described and identified above.

The example of embodiment illustrated in Figure 3 exploits the release of the two drugs in different times. In the case where the first layer is constituted by Paclitaxel and the second layer is constituted by FK506, there will initially be an effect (which is likely to be of an anti-inflammatory type) on account of the release of FK506 and only subsequently an effect (which is likely to be of an anti-proliferative type) linked to the release of Paclitaxel. In the case where it were desired to favour initially the anti-proliferative effect and only subsequently an anti-inflammatory effect for promoting final healing of the blood vessel, the first layer 2 could be advantageously constituted by FK506 and the second layer 3 by Paclitaxel, it being evident that it is always the second layer 3 positioned within the cavity 12 to be absorbed first by the walls of the vessel. Even though in this case a mixture of the two drugs is not used, there is even so obtained a combined (therapeutic) effect of the two drugs in the implantation site.

This example of embodiment involves depositing, in the form of powder or paste, one of the two drugs or a mixture thereof on the bottom of the cavity, followed by a step of stablization of this first layer; only subsequently, is the second drug or mixture of drugs deposited and then stabilized. In the case where stablization envisages the use of a solvent, there could be hypothesized the use of two different solvents for stablization of the two layers for the purpose of preventing undesirable mixing of the two drugs at the interface of contact between the two layers.

In the case where it is necessary, rather than appropriate, to envisage a non-immediate action of the two drugs after implantation of the stent or else at times regulated with greater precision, said controlled release can be obtained via the introduction of layers constituted by polymers having a porous structure, or by bio-erodible polymers, or by excipient substances.

Figure 4a illustrates a case where the aim is that the layer of mixture 5 set within the cavity should be made available to the organism after a definite period of time t subsequent to the moment of implantation of the stent (understood as time zero). In these cases, the layer of polymer or of excipient substance 4 is applied so as to be eroded/absorbed by the wall of the vessel in a time t determined by the composition as well as by the thickness of said layer 4. Only subsequently will the mixture 5 of two drugs be released. Figure 4b shows the application of the same principle to a solution in which the two drugs 2 and 3 (whichever the drug constituting each layer) are arranged on top of one another.

A polymeric layer or a layer of excipient substances 4 can moreover be used, with the same modalities, for the physical separation of the two layers of drugs 2 and 3 (see Figures 5a and 5b) in the case where it is desired to prevent their spontaneous mixing at the interface of contact of the two layers. An example of application of said solution can be represented by the case where the same solvent is used for the stablization of the two drugs deposited within the cavity in two successive steps, where said identity of solvent could favour the destabilization of a surface portion of the layer of a drug already present in the cavity, with subsequent mixing with the powder or paste of the second drug not yet stabilized.

As regards the composition of the bioerodible-polymer-based layer 4, reference can be made to the very extensive literature published on the subject, which is well known to the person skilled in the branch. By way of example it is possible to cite as suitable polymers: acrylates and methacrylates, silicones, such as for example polydimethylsiloxane, polymethylene malonester, polyethers, polyesters, bioabsorbable polymers, polymers of vinylic monomers, such as for example polyvinyl pyrrolidone and vinyl ether, poly-cis-1,4-butadiene, poly-cis-1,4-isoprene, poly-trans-1,4-isoprene and vulcanized products, polyurethane, polycarbamides, polyamides, polysulphones and biopolymers, such as for example cellulose and its derivatives, proteins, and fibrin bonding agents. Particularly interesting properties are demonstrated by hydrogels, which on account of their high absorption of water have very good haemocompatiblity as outermost layer (top coat). It is, for example, possible to use hydrogels such as polyacrylamide, polyacrylic acid, polymers with oxygen as heteroatom in the main chain, such as for example polyethylene oxide, polypropylene oxide, polytetrahydrofuran.

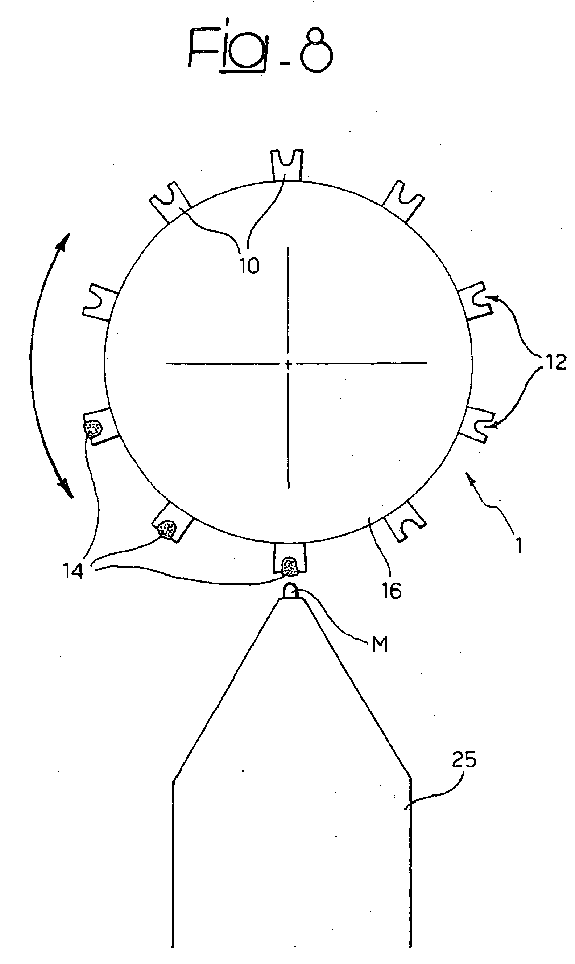

Figure 6 envisages applying homogeneously on the entire outer surface of the stent (and not only within the cavities 12) a layer of coating 6 constituted by the mixture of the two drugs or else by just one of the two drugs for the purpose of increasing substantially the therapeutic impact *in situ* on the biological processes that underlie restenosis.

The top coat 6 of mixture of the two drugs or of just one of the drugs is performed subsequent to filling of the cavities, once again using a powder or paste of the mixture or of the single drug of interest. The modalities of loading and stablization of the layer of top coat 6 are similar to the ones previously described in relation to the loading of the cavities. In principle, this top coat 6 of the stent could be applied when the stent has already been positioned on the catheter for implantation for the purpose of preventing problems linked to the manipulation of the coated stent during assembly of the stent on the catheter and hence preventing any problem of damage to the coating, with possible loss of a portion thereof. It is evident that the quantity of the two drugs loaded/applied on the stent as illustrated in Figure 6 always respect the weight ratios rather than the total or individual amounts of the two drugs with respect to one other, as described above.

Illustrated in Figure 7 is an example of embodiment in which the cavity 12 in the stent 1 has a step-like profile, i.e., a configuration such as to provide further degrees of freedom in the choice of: i) the quantity of a drug/mixture to be introduced within the cavity; and ii) the kinetics of release of the drug/mixture, which is obviously controlled by the surface of opening of the cavity.

Figure 8 refers to the possibility of applying selectively, and hence only within the cavities 12, respective amounts 14 of material in the form of paste by means of a dispensing nozzle 25, which may be brought into a position facing the cavities 12, bestowing then upon the cavity 12 each time involved and upon the nozzle 25 a relative displacement that leads the nozzle to "scan" the cavity 12, depositing therein the drug/mixture M.

This solution may be implemented with high precision by resorting to a numeric-control machine for controlling the relative movement (usually of translation and rotation) of the support 16 that carries the stent 1 and the dispensing nozzle 25.

Once again without wishing to be tied down to any specific theory in this connection, the present applicant has reason to believe that the choice of the values of the Paclitaxel:FK506 weight ratio in the ranges from 1:72 to 1:0.2, preferably from 1:18 to 1:0.2, more preferably from 1:8 to 1:0.5, still more preferably from 1:2 to 1:0.7, and to an even more preferred extent from 1:3 to 1:1, is particularly beneficial.

Without prejudice to the principle of the invention, the details of implementation and the embodiments may vary with respect to what is described and illustrated herein, without thereby departing from the scope of the invention, as defined by the annexed claims.

## Claims

1. A stent (1) comprising a radially expandable body, provided with formations for receiving agents for the treatment of the site of implantation of the stent, wherein said agents for treatment are constituted by a combination of Paclitaxel and FK506 or their **derivatives or analogues characterized in that** said agents are present in a Paclitaxel:FK506 weight ratio with respect to the total quantity of said agents applied on said stent comprised in the range from 1:72 to 1:0.2..

2. The stent according to Claim 1, **characterized in that** said formations for reception are constituted by cavities (12) having an opening towards the outer surface of said stent.

3. The stent according to any one of the preceding claims, **characterized in that** said stent is formed by elements (struts) defining a reticular structure.

4. The stent according to any one of the preceding claims, **characterized in that** said formations for reception are present on said elements.

5. The stent according to Claim 1, **characterized in that** said weight ratio is comprised in the range from 1:18 to 1:0.2.

6. The stent according to Claim 1, **characterized in that** said weight ratio is comprised in the range from 1:8 to 1:0.5.

7. The stent according to Claim 1, **characterized in that** said weight ratio is comprised in the range from 1:2 to 1:0.7.

8. The stent according to Claim 1, **characterized in that** said weight ratio is comprised in the range from 1:3 to 1:1.

9. The stent according to any one of the preceding claims, **characterized in that** said agents are present in a total quantity comprised in the range between 50 and 1000 micrograms.

10. The stent according to Claim 9, **characterized in that** said quantity is comprised in the range between 70 and 700 micrograms.

11. The stent according to Claim 9, **characterized in that** said quantity is comprised in the range between 140 and 500 micrograms.

12. The stent according to Claim 9, **characterized in that** said quantity is comprised in the range between 200 and 400 micrograms.

13. The stent according to any one of the preceding claims, **characterized in that** Paclitaxel is present in a quantity by weight of at least 25 micrograms.

14. The stent according to Claim 13, **characterized in that** Paclitaxel is present in a quantity by weight of at least 80 micrograms.

15. The stent according to any one of the preceding claims, **characterized in that** FK506 is present in a quantity by weight of at least 60 micrograms.

16. The stent according to Claim 15, **characterized in that** FK506 is present in a quantity by weight of at least 135 micrograms.

17. The stent according to any one of the preceding claims, **characterized in that** said formations for reception are constituted by cavities (12) and that said cavities (12) contain a homogeneous mixture (5) of said agents.

18. The stent according to any one of the preceding claims, **characterized in that** said agents are arranged in a layered structure comprising at least two layers (2, 3).

19. The stent according to Claim 18, **characterized in that** said layers are homogeneously constituted by: just one of said agents; a mixture of said agents.

20. The stent according to Claim 18, **characterized in that** associated to said layered structure is a layer (4), said associated layer (4) comprising at least one between a polymeric material and an excipient substance.

21. The stent according to Claim 20, **characterized in that** said associated layer (4) is situated in an internal position of said structure.

22. The stent according to Claim 21, **characterized in that** said associated layer (4) is situated in a position external to said structure and with respect to said stent (1).

23. The stent according to any one of the preceding claims, **characterized in that** said stent has a top coat (6) constituted by one of said agents or by said mixture of said agents.

24. The stent according to any one of the preceding claims, **characterized in that** said formations for reception are constituted by cavities (12) and **in that** said cavities (12) have a substantially step-like profile.

25. The stent according to any one of the preceding claims, **characterized in that** said agents are applied in the form of powders or pastes obtained from said powders.

26. The stent according to any one of the preceding claims, **characterized in that** added to said agents are pharmacologically acceptable excipients/additives

## Patentansprüche

1. Ein Stent (1) mit einem radial aufweitbaren Körper, welcher mit Formationen für die Aufnahme von Substanzen für die Behandlung des Implantationsbereichs des Stentes versehen ist, worin die Substanzen für die Behandlung von einer Kombination aus Paclitaxel und FK506 oder deren Derivaten oder Analoga gebildet werden, **dadurch gekennzeichnet, dass** die Substanzen in einem Paclitaxel: FK506-Gewichtsverhältnis in dem Bereich von 1:72 bis 1:0,2 mit Bezug auf die Gesamtmenge der auf den Stents verwendeten Substanzen vorhanden sind.

2. Der Stent gemäß Anspruch 1, **dadurch gekennzeichnet, dass** die Formationen für die Aufnahme von Hohlräumen (12) gebildet werden, welche eine Öffnung in Richtung der äußeren Oberfläche des Stents aufweisen.

3. Der Stent gemäß einem der vorhergehenden Ansprüche, **dadurch gekennzeichnet, dass** der Stent von Elementen (Streben) gebildet ist, die eine netzförmige Struktur definieren.

4. Der Stent gemäß einem der vorhergehenden Anspruch, **dadurch gekennzeichnet, dass** die Formationen für die Aufnahme auf den Elementen vorhanden sind.

5. Der Stent gemäß Anspruch 1, **dadurch gekennzeichnet, dass** das Gewichtsverhältnis in dem Bereich von 1:18 bis 1:0,2 liegt.

6. Der Stent gemäß Anspruch 1, **dadurch gekennzeichnet, dass** das Gewichtsverhältnis in dem Bereich von 1:8 bis 1:0,5 liegt.

7. Der Stent gemäß Anspruch 1, **dadurch gekennzeichnet, dass** das Gewichtsverhältnis in dem Bereich von 1:2 bis 1:0,7 liegt.

8. Der Stent gemäß Anspruch 1, **dadurch gekennzeichnet, dass** das Gewichtsverhältnis in dem Bereich von 1:3 bis 1:1 liegt.

9. Der Stent gemäß einem der vorhergehenden Ansprüche, **dadurch gekennzeichnet, dass** die Substanzen in einer Gesamtmenge in dem Bereich zwischen 50 bis 1000 Mikrogramm vorhanden sind.

10. Der Stent gemäß Anspruch 9, **dadurch gekennzeichnet, dass** die Menge in dem Bereich zwischen 70 bis 700 Mikrogramm liegt.

11. Der Stent gemäß Anspruch 9, **dadurch gekennzeichnet, dass** die Menge in dem Bereich zwischen 140 bis 500 Mikrogramm liegt.

12. Der Stent gemäß Anspruch 9, **dadurch gekennzeichnet, dass** die Menge in dem Bereich zwischen 200 bis 400 Mikrogramm liegt.

13. Der Stent gemäß einem der vorhergehenden Ansprüche, **dadurch gekennzeichnet, dass** Paclitaxel in einer Menge bezogen auf das Gewicht von mindestens 25 Mikrogramm vorhanden ist.

14. Der Stent gemäß Anspruch 13, **dadurch gekennzeichnet, dass** Palitaxel in einer Menge bezogen auf das Gewicht von mindestens 80 Mikrogramm vorhanden ist.

15. Der Stent gemäß einem der vorhergehenden Ansprüche, **dadurch gekennzeichnet, dass** FK506 in einer Menge bezogen auf das Gewicht von mindestens 60 Mikrogramm vorhanden ist.

16. Der Stent gemäß Anspruch 15, **dadurch gekennzeichnet, dass** FK506 in einer Menge bezogen auf das Gewicht von mindestens 135 Mikrogramm vorhanden ist.

17. Der Stent gemäß einem der vorhergehenden Ansprüche, **dadurch gekennzeichnet, dass** die Formationen für die Aufnahme von Hohlräumen (12) gebildet werden und dass die Hohlräume (12) eine homogene Mischung (5) der Substanzen enthalten.

18. Der Stent gemäß einem der vorhergehenden Ansprüche, **dadurch gekennzeichnet, dass** die Substanzen in einer schichtartigen Struktur angeordnet sind, welche mindestens zwei Schichten (2, 3) aufweist.

19. Der Stent gemäß Anspruch 18, **dadurch gekennzeichnet, dass** die Schichten homogen gebildet werden von: nur einer der Substanzen; einer Mischung der Substanzen.

20. Der Stent gemäß Anspruch 18, **dadurch gekennzeichnet, dass** der Schichtstruktur eine Schicht (4) zugeordnet ist, wobei die zugeordnete Schicht (4) zumindest eine von einem Polymermaterial und einer Arzneiträgerstoffsubstanz umfaßt.

21. Der Stent gemäß Anspruch 20, **dadurch gekennzeichnet, dass** die zugeordnete Schicht (4) in einer inneren Position der Struktur angeordnet ist.

22. Der Stent gemäß Anspruch 21, **dadurch gekennzeichnet, dass** die zugeordnete Schicht (4) in einer äußeren Position bezüglich der Struktur und mit Bezug auf den Stent (1) angeordnet ist.

23. Der Stent gemäß einem der vorhergehenden Ansprüche, **dadurch gekennzeichnet, dass** der Stent einen Deckanstrich (6) aufweist, welcher aus einer der Substanzen oder aus einer Mischung der Substanzen gebildet ist.

24. Der Stent gemäß einem der vorhergehenden Ansprüche, **dadurch gekennzeichnet, dass** die Formationen für die Aufnahme von Hohlräumen (12) gebildet werden und dass die Hohlräume (12) im wesentlichen ein stufenförmiges Profil aufweisen.

25. Der Stent gemäß einem der vorhergehenden Ansprüche, **dadurch gekennzeichnet, dass** die Substanzen in der Form von Pulvern oder Pasten, welche aus den Pulvern erhalten werden, aufgebracht werden.

26. Der Stent gemäß einem der vorhergehenden Ansprüche, **dadurch gekennzeichnet, dass** zu den Substanzen pharmakologisch zulässige Arzneiträgerstoffe und Zusätze hinzugefügt sind.

## Revendications

1. Stent (1) comprenant un corps radialement expansible, prévu avec des formations pour recevoir des agents pour le traitement du site d'implantation du stent, dans lequel les agents pour le traitement sont constitués par une combinaison de Paclitaxel et FK506 ou leurs dérivés ou analogues, **caractérisé en ce que** lesdits agents sont présents dans un rapport pondéral Paclitaxel:FK506 par rapport à la quantité totale desdits agents appliqués sur ledit stent compris entre 1:72 à 1:0,2.

2. Stent selon la revendication 1, **caractérisé en ce que** lesdites formations pour la réception sont constituées par des cavités (12) ayant une ouverture vers la surface externe dudit stent.

3. Stent selon l'une quelconque des revendications précédentes, **caractérisé en ce que** ledit stent est formé par des éléments (entretoises) définissant une structure réticulaire.

4. Stent selon l'une quelconque des revendications précédentes, **caractérisé en ce que** lesdites formations pour la réception sont présentes sur lesdits éléments.

5. Stent selon la revendication 1, **caractérisé en ce que** ledit rapport pondéral est compris entre 1:18 et 1:0,2.

6. Stent selon la revendication 1, **caractérisé en ce que** ledit rapport pondéral est de l'ordre de 1:8 à 1:0,5.

7. Stent selon la revendication 1, **caractérisé en ce que** ledit rapport pondéral est de l'ordre de 1:2 à 1:0,7.

8. Stent selon la revendication 1, **caractérisé en ce que** ledit rapport pondéral est de l'ordre de 1:3 à 1:1.

9. Stent selon l'une quelconque des revendications précédentes, **caractérisé en ce que** lesdits agents sont présents dans une quantité totale comprise entre 50 et 1 000 microgrammes.

10. Stent selon la revendication 9, **caractérisé en ce que** ladite quantité est comprise entre 70 et 700 microgrammes.

11. Stent selon la revendication 9, **caractérisé en ce que** ladite quantité est comprise entre 140 et 500 microgrammes.

12. Stent selon la revendication 9, **caractérisé en ce que** ladite quantité est comprise entre 200 et 400 microgrammes.

13. Stent selon l'une quelconque des revendications précédentes, **caractérisé en ce que** le Paclitaxel est présent dans une quantité en poids d'au moins 25 microgrammes.

14. Stent selon la revendication 13, **caractérisé en ce que** le Paclitaxel est présent dans une quantité en poids d'au moins 80 microgrammes.

15. Stent selon l'une quelconque des revendications précédentes, **caractérisé en ce que** le FK506 est présent dans une quantité en poids d'au moins 60 microgrammes.

16. Stent selon la revendication 15, **caractérisé en ce que** le FK506 est présent dans une quantité en poids d'au moins 135 microgrammes.

17. Stent selon l'une quelconque des revendications précédentes, **caractérisé en ce que** lesdites formations pour la réception sont constituées par des cavités (12) et **en ce que** lesdites cavités (12) contiennent un mélange homogène (5) desdits agents.

18. Stent selon l'une quelconque des revendications précédentes, **caractérisé en ce que** lesdits agents sont agencés dans une structure en couches comprenant au moins deux couches (2, 3).

19. Stent selon la revendication 18, **caractérisé en ce que** lesdites couches sont constituées de manière homogène par un seul desdits agents ; un mélange desdits agents.

20. Stent selon la revendication 18, **caractérisé en ce que** associée à ladite structure en couche, on trouve une couche (4), ladite couche associée (4) comprenant au moins l'un parmi un matériau polymère et une substance d'excipient.

21. Stent selon la revendication 20, **caractérisé en ce que** ladite couche (4) associée est située dans une position interne de ladite structure.

22. Stent selon la revendication 21, **caractérisé en ce que** ladite couche associée (4) est située dans une position externe par rapport à ladite structure et par rapport audit stent (1).

23. Stent selon l'une quelconque des revendications précédentes, **caractérisé en ce que** ledit stent a un revêtement supérieur (6) constitué par l'un desdits agents ou par ledit mélange desdits agents.

24. Stent selon l'une quelconque des revendications précédentes, **caractérisé en ce que** lesdites formations pour la réception sont constituées par des cavités (12) et **en ce que** lesdites cavités (12) ont un profil sensiblement en forme de marche.

25. Stent selon l'une quelconque des revendications précédentes, **caractérisé en ce que** lesdits agents sont appliqués sous forme de poudres ou de pâtes obtenues à partir desdites poudres.

26. Stent selon l'une quelconque des revendications précédentes, **caractérisé en ce que** ajoutés auxdits agents, on trouve des excipients/additifs pharmacologiquement acceptables.
